# EUROPÄISCHE PATENTSCHRIFT

(11) **EP 2 791 659 B1**
(45) Veröffentlichungstag und Bekanntmachung des Hinweises auf die Patenterteilung: **23.12.2020**
(21) Anmeldenummer: 12788446.8
(22) Anmeldetag: 16.11.2012
(51) Int. Cl.: G01N 21/78, G01N 33/18, G01N 21/31

(54) **KÜVETTE UND VERFAHREN ZUR BESTIMMUNG DES CHEMISCHEN SAUERSTOFFBEDARFS**
CUVETTE AND METHOD FOR DETERMINING THE CHEMICAL OXYGEN DEMAND
CUVETTE ET PROCÉDÉE POUR DÉTERMINER LA DEMANDE CHIMIQUE EN OXYGÈNE

(30) Priorität: 12.12.2011 EP 11009778
(43) Veröffentlichungstag der Anmeldung: 22.10.2014
(73) Patentinhaber: Merck Patent GmbH, 64293 Darmstadt (DE)
(72) Erfinder: OLT, Ralf, 64750 Luetzelbach-Seckmauern (DE); DECKER, Gunter, 64625 Bensheim (DE)
(86) Internationale Anmeldenummer: PCT/EP2012/004761
(87) Internationale Veröffentlichungsnummer: WO 2013/087143

(56) Entgegenhaltungen:
- CN-A- 101 450 829
- CN-Y- 2 720 437
- DE-A1- 2 022 640
- US-A1- 2011 027 893

## Beschreibung

Die Erfindung betrifft ein Mittel und ein Verfahren zur photometrischen Bestimmung des chemischen Sauerstoffbedarfs von Proben, die Chlorid enthalten.

Der Chemische Sauerstoffbedarf (CSB, engl. chemical oxygen demand, COD) ist als Summenparameter ein Maß für die Summe aller im Wasser vorhandenen, unter bestimmten Bedingungen oxidierbaren Stoffe. Er gibt die Menge an Sauerstoff (in mg/l) an, die zu ihrer Oxidation benötigt würde, wenn Sauerstoff das Oxidationsmittel wäre. Das Verfahren wird auch unter der Bezeichnung "Oxidierbarkeit Cr-VI" (Chromat-Verbrauch, wenn dieses das Oxidationsmittel wäre) dem Kaliumpermanganatverbrauch ("Oxidierbarkeit Mn-VII") gegenüber gestellt. Neben dieser Verwendung als Maß für die Konzentration chemisch oxidierbarer Stoffe im Wasser, wird der Chemischer Sauerstoffbedarf auch als Maß der chemisch oxidierbaren Stoffe, die bei der Produktion einer Produktmenge ins Abwasser abgegebenen (g/kg Produktmenge) oder die in einem Zeitraum entsorgt werden (t/a, Tonnen pro Jahr), verwendet.

Zur Ermittlung des CSB wird eine Wasserprobe mit Schwefelsäure stark angesäuert und mit einer vorgegebenen genauen Menge des starken Oxidationsmittels Kaliumdichromat (K₂Cr₂O₇) unter Zusatz von Silbersulfat als Katalysator erhitzt.

DIN 38409-41 (auch DEV H41) beschreibt die CSB-Bestimmung mittels Titration. Diese Norm beschreibt unter Methode 41-1 ein titrimetrisches Verfahren für Proben mit Chlorid-Gehalt bis zu 1000 mg/l.
Unter Methode 41-2 ist ein Verfahren zur Abreicherung des Chlorid-Gehalts von Proben mit einem Chlorid-Gehalt über 1000 mg/l beschrieben.

Nach Abreicherung kann wie unter 41-1 beschreiben der CSB mittels Titration bestimmt werden.

Vor allem zur Überwachung des CSB auf Kläranlagen und anderen wassertechnischen Anlagen, in denen kein Labor und geschultes Laborpersonal verfügbar ist, erfolgt die CSB-Bestimmung jedoch meist mittels sogenannter Küvetten-Tests (Schnellmethoden). Diese Test-Kits können auch bei geringen Vorkenntnissen angewendet werden, enthalten bereits alle notwendigen Reagenzien und erfordern nur wenige Laborgeräte. Bei diesem Verfahren erfolgt die Bestimmung des Dichromat-Verbrauchs nicht titrimetrisch sondern photometrisch.

Ein CSB-Test, der in einem Proberöhrchen durchgeführt wird, ist beispielsweise aus dem chinesischen Gebrauchsmuster CN2720437Y bekannt.

Die DIN ISO 15705 beschreibt die photometrische Bestimmung des CSB mittels des Küvettentests. Jedoch ist diese Bestimmung auf Proben mit einem Chlorid-Gehalt von max. 1000 mg/l eingeschränkt.

Es existieren CSB Küvettentests verschiedener Anbieter, die durch Variation des Probe-Reagenz-Verhältnisses und einer Erhöhung der Quecksilber(II)sulfat Menge in den Reagenzien, eine Bestimmung von Proben mit einem Chlorid-Gehalt bis zu 5000 mg/l bei einem gleichzeitigen CSB-Gehalt von bis zu 2000 mg/l ermöglichen.

Auch ist durch eine Vorverdünnung der Proben mit dest. Wasser, sowie einer Verdünnung der Proben mit Reagenzien eine höhere Chloridtoleranz zu erzielen, jedoch wird dabei auch der CSB-Gehalt der Probe mitverdünnt. Somit ist diese Vorgehensweise nicht für Proben mit niedrigem CSB-Gehalt geeignet.

Eine Probe mit einem CSB Gehalt von unter 2000 mg/l und einem Chlorid-Gehalt über 5000 mg/l kann bislang nicht mittels eines Küvettentests analysiert werden. Hierfür steht nur das wesentlich aufwändigere Titrationsverfahren zur Verfügung. Grund für diese Einschränkung ist, dass Proben mit einem Chlorid-Gehalt von über 5000 mg/l zunächst nach DIN 38409-41, Methode 41-2 abgereichert werden müssen. Dazu werden nach der Norm 20 ml der Analysenprobe mit 25 ml konzentrierter Schwefelsäure versetzt und mehrere Stunden gerührt. Das Chlorid wird so als Chlorwasserstoff ausgetrieben. Die dadurch erhaltene schwefelsaure Probe ist für die Bestimmung des CSB mittels des Küvettentests nach DIN ISO 15705 nicht geeignet.

Dies stellt jedoch eine starke Einschränkung der Anwendbarkeit des Küvettentests dar, da beispielsweise Chlorid-haltige Industrieabwässer oder Meerwasserproben bislang nicht mittels eines Küvettentests nach DIN ISO 15705 untersucht werden können.

Aufgabe der vorliegenden Erfindung war es daher, einen Küvettentest bevorzugt mit einfachen, gebrauchsfertigen Reagenzien zur Verfügung zu stellen, der auch für Ausgangs-Proben mit einem CSB Gehalt von unter 2000 mg/l und einem Chlorid-Gehalt über 5000 mg/l geeignet ist. Diese Proben sind nach der Chlorid-Abreicherung für die CSB-Bestimmung stark Schwefelsäure-haltig.

Es wurde nun gefunden, dass eine bestimmte Zusammensetzung der Reagenzlösung für den Küvettentest diesen auch für die Bestimmung des CSB in Chlorid-abgereicherten Proben zugänglich macht. Darüber hinaus wurde gefunden, dass die erfindungsgemäße Reagenzlösung erstmals die photometrische Bestimmung des CSB-Gehalts von z.B. nach DIN 38409-41-2 Chlorid-abgereicherten Proben mit einer Reagenzlösung ermöglicht, die kein toxisches Quecksilber(II)sulfat enthält.

Gegenstand der vorliegenden Erfindung ist daher eine Küvette enthaltend eine Reagenzlösung zumindest bestehend aus
- Kaliumdichromat (K₂Cr₂O₇)
- Silbersulfat (Ag₂SO₄)
- Wasser
- Schwefelsäure
dadurch gekennzeichnet, dass der Gehalt an Schwefelsäure zwischen 20 und 50% (w/w) beträgt. Die Reagenzlösung der vorliegenden Erfindung ist in Anspruch 1 definiert.

In einer bevorzugten Ausführungsform ist die Küvette reversibel verschließbar.

In einer Ausführungsform enthält die Reagenzlösung zusätzlich Quecksilber(II)sulfat (HgSO₄).

In einer bevorzugten Ausführungsform besteht die Reagenzlösung nur aus 0,1 bis 2,5 Gewichts% Kaliumdichromat (K₂Cr₂O₇), 0,1 bis 2 Gewichts% Silbersulfat (Ag₂SO₄), 20 bis 50 Gewichts% Schwefelsäure und Wasser.

In einer bevorzugten Ausführungsform ist die Reagenzlösung hergestellt durch Mischen von mindestens einer Lösung, die zumindest

Kaliumdichromat und Wasser, aber kein Silbersulfat und kein Quecksilber(II)sulfat enthält und einer Lösung, die zumindest Silbersulfat und Schwefelsäure, aber kein Quecksilbersulfat und kein Kaliumdichromat enthält.

Gegenstand der vorliegenden Erfindung ist auch ein Verfahren zur Bestimmung des CSB-Gehalts in Chlorid-haltigen Proben durch
a) Austreiben der Chlorid-Ionen aus der Probe durch Behandlung der Probe mit Schwefelsäure
b) Mischen zumindest eines Teils der aus Schritt a) erhaltenen Probe mit einer Reagenzlösung zumindest bestehend aus
   - Kaliumdichromat (K₂Cr₂O₇)
   - Silbersulfat (Ag₂SO₄)
   - optional Quecksilber(II)sulfat (HgSO₄)
   - Wasser
   - Schwefelsäure
   wobei der Gehalt an Schwefelsäure zwischen 20 und 50 %(w/w) beträgt
c) Inkubation der Mischung aus Schritt b) für mindestens 20 Minuten bei einer Temperatur über 100°C.
d) photometrische Bestimmung des CSB Gehalts der Mischung aus c). Das erfindungsgemäße Verfahren ist in Anspruch 6 definiert.

In einer bevorzugten Ausführungsform werden die Verfahrensschritte b) bis d) in einer reversibel verschließbaren Küvette durchgeführt.

In einer bevorzugten Ausführungsform erfolgt das Austreiben in Schritt a) mit konzentrierter Schwefelsäure.

In einer bevorzugten Ausführungsform erfolgt die Inkubation in Schritt c) für 20 bis 150 Minuten bei einer Temperatur zwischen 140 und 180°C.

In einer bevorzugten Ausführungsform werden in Schritt b) die Probe und die Reagenzlösung in einem Volumenverhältnis Probe : Reagenzlösung zwischen 5:1 und 2:1 gemischt.

In einer bevorzugten Ausführungsform besteht die Reagenzlösung nur aus 0,1 bis 2,5 Gewichts% Kaliumdichromat (K₂Cr₂O₇), 0,1 bis 2 Gewichts% Silbersulfat (Ag₂SO₄), 20 bis 50 Gewichts% Schwefelsäure und Wasser.

In einer bevorzugten Ausführungsform ist die Reagenzlösung hergestellt durch Mischen von mindestens einer Lösung, die zumindest Kaliumdichromat und Wasser, aber kein Silbersulfat und kein Quecksilber(II)sulfat enthält und einer Lösung, die zumindest Silbersulfat und Schwefelsäure, aber kein Quecksilbersulfat und kein Kaliumdichromat enthält.

In einer bevorzugten Ausführungsform erfolgt die photometrische Bestimmung in Schritt c) durch Messung der Extinktion bei einer Wellenlänge im Bereich von 300 - 700 nm.

In einer weiteren bevorzugten Ausführungsform wird Schwefelsäure mit einem CSB-Gehalt von weniger als 5 mg/l für die Herstellung der Reagenzlösung eingesetzt.

In einer weiteren bevorzugten Ausführungsform wird Schwefelsäure mit einem CSB-Gehalt von weniger als 5 mg/l für die Chlorid-Abreicherung bevorzugt durchgeführt nach DIN 38409-41-2 eingesetzt.

Eine Küvette ist erfindungsgemäß ein Gefäß, in dem photometrische Messungen durchgeführt werden können. Typischerweise bestehen Küvetten aus Quarz, Glas oder Kunststoff und haben mindestens zwei planparallele Seitenflächen oder sind rund. Bevorzugt sind Rundküvetten. Bevorzugt sind zudem erfindungsgemäß reversibel verschließbare Küvetten. Diese reversibel und bevorzugt gasdicht verschließbaren Küvetten können direkt als Gefäß zur Durchführung des erfindungsgemäßen Verfahrens verwendet werden. Dazu können die Küvetten bereits mit der Reagenzlösung befüllt und gelagert werden. Dann kann die Probe zugeben werden und die Küvette für die Inkubation und die nachfolgenden Verfahrensschritte wieder fest verschlossen werden.

Um den CSB zuverlässig und vergleichbar zu bestimmen, müssen möglichst konstante Reaktionsbestimmungen vorliegen. Diese Reaktionsbedingungen werden beispielsweise von den DIN Normen DIN 38409-41 und DIN ISO 15705 vorgegeben. Dem Fachmann ist die Durchführung einer CSB-Bestimmung generell bekannt. Einzelheiten sind den DIN-Normen zu entnehmen.

Auch das erfindungsgemäße Verfahren ist bei Einhaltung konstanter Reaktionsbedingungen zur Bestimmung des CSB mit einer den DIN-Normen vergleichbaren Empfindlichkeit und Konstanz geeignet.

Während Proben mit einem Chlorid-Gehalt über 5000 mg/l bisher zuverlässig nur titrimetrisch vermessen werden konnten, kann dies erfindungsgemäß nun auch photometrisch erfolgen. Es wurde gefunden, dass eine Verringerung des Gehalts an Schwefelsäure in der Reagenzlösung auch Proben mit einem hohen Schwefelsäuregehalt der photometrischen Messung zugänglich macht. Bislang wurde es als schwierig angesehen, Reagenzlösungen mit einem geringeren Gehalt an Schwefelsäure zuverlässig herzustellen, da die Löslichkeiten von Kaliumdichromat (K₂Cr₂O₇), Silbersulfat (Ag₂SO₄) und Quecksilber(II)sulfat sich mit der Veränderung des Schwefelsäuregehalts auch stark verändern.

Die erfindungsgemäße Reagenzlösung enthält zumindest Kaliumdichromat (K₂Cr₂O₇), Silbersulfat (Ag₂SO₄), Wasser und Schwefelsäure, wobei der Gehalt an Schwefelsäure zwischen 20 und 50% (w/w) liegt.
In einer bevorzugten Ausführungsform liegt der Gehalt an Kaliumdichromat (K₂Cr₂O₇) in der Reagenzlösung zwischen 0,1 bis 2,5 Gewichts% und der Gehalt an Silbersulfat (Ag₂SO₄) zwischen 0,1 bis 2 Gewichts%.

Optional kann die Reagenzlösung auch Quecksilber(II)sulfat enthalten.

In einer besonders bevorzugten Ausführungsform besteht die Reagenzlösung nur aus 0,1 bis 2,5 Gewichts% Kaliumdichromat (K₂Cr₂O₇), 0,1 bis 2 Gewichts% Silbersulfat (Ag₂SO₄), 20 bis 50 Gewichts% Schwefelsäure und Wasser.

Es wurde nun gefunden, dass man besonders reproduzierbare Ergebnisse mit erfindungsgemäßen Reagenzlösungen erhält, die zumindest Kaliumdichromat (K₂Cr₂O₇), Silbersulfat (Ag₂SO₄), Schwefelsäure und Wasser sowie optional Quecksilber(II)sulfat enthalten und durch Mischung von mindestens zwei Einzellösungen hergestellt wurden. Dabei enthält eine Einzellösung zumindest Kaliumdichromat und Wasser, aber kein Silbersulfat und kein Quecksilber(II)sulfat. Die andere Einzellösung enthält zumindest Silbersulfat und Schwefelsäure, aber kein Quecksilbersulfat und kein Kaliumdichromat. Besonders bevorzugt enthält diese Einzellösung Silbersulfat und konzentrierte Schwefelsäure. Enthält die Reagenzlösung zusätzlich Quecksilbersulfat, so wird eine dritte Einzellösung hergestellt, die zumindest Quecksilber(II)sulfat, Wasser und Schwefelsäure enthält, aber kein Silbersulfat und kein Kaliumdichromat. Besonders bevorzugt enthält diese dritte Einzellösung Quecksilber(II)sulfat in 5 bis 25%iger Schwefelsäure (v/v).

Bevorzugte Zusammensetzungen der Einzellösungen zur Herstellung der erfindungsgemäßen Reagenzlösung sind:
- zwischen 10 und 20% (w/w) Hg-Sulfat in verdünnter wässriger Schwefelsäure (zwischen 10 und 20% (w/w))
- zwischen 2,5 und 7,5 % (w/w) Silbersulfat in konzentrierter Schwefelsäure (92% (w/w))
- zwischen 2,5 und 5 % (w/w) Kaliumdichromat in Wasser

Besonders bevorzugte Zusammensetzungen sind:
- Hg-Sulfat 15,5% (w/w) in verdünnter Schwefelsäure 14,5% (w/w)
- Silbersulfat 4,3% (w/w) in Schwefelsäure 92% (w/w)
- Kaliumdichromat 3,6% (w/w) in Wasser

Die Einzellösungen werden dann zur Herstellung der erfindungsgemäßen Reagenzlösung gemischt.

Die erfindungsgemäße Reagenzlösung ist insbesondere geeignet zur Bestimmung des CSB in Proben, bei denen zuvor die Chlorid-Ionen durch Behandlung mit Schwefelsäure ausgetrieben wurden. Dem Fachmann ist das Verfahren zur Austreibung von Chlorid-Ionen mit Schwefelsäure bekannt. Details finden sich in DIN 38409-41, Methode 41-2. Wird das Verfahren zur Austreibung von Chlorid entsprechend der Vorschriften der DIN 38409-41, Methode 41-2 durchgeführt, wird typischerweise eine Abreicherung bis zu einem Rest-Chlorid-Gehalt von maximal 2 ppm erreicht.

Üblicherweise wird das Verfahren zur Austreibung von Chlorid mit Schwefelsäure bisher nur bei Proben mit einem Chlorid-Gehalt über 1000 mg/l angewendet. Proben mit einem Chlorid-Gehalt unter 1000 mg/l werden üblicherweise mit einer Reagenzlösung behandelt, die Quecksilber(II)sulfat enthält. Quecksilber(II)sulfat maskiert in der Probe vorhandene Chlorid-ionen. Quecksilber(II)sulfat ist jedoch sehr toxisch und umweltschädlich. Daher sollte der Einsatz von Quecksilber(II)sulfat soweit möglich vermieden werden.

Das erfindungsgemäße Verfahren bietet nun die Möglichkeit, den CSB von Chlorid-haltigen Proben photometrisch zu bestimmen, ohne Quecksilber(II)sulfat zu verwenden. Dazu wird die Chlorid-haltige Probe - unabhängig davon, ob sie einen Chlorid-Gehalt über oder unter 1000 mg/l aufweist - zunächst zum Austreiben der Chlorid-Ionen mit Schwefelsäure behandelt. Anschließend erfolgt die photometrische Bestimmung des CSB mit der erfindungsgemäßen Reagenzlösung. Ein Zusatz von Quecksilber(II)sulfat zu der Reagenzlösung ist in der Regel nicht notwendig, da das Austreiben mit Schwefelsäure den Chlorid-Gehalt der Probe so stark senkt, dass das in geringsten Mengen in der Probe verbleibende Chlorid die weitere Messung nicht beeinträchtigt.

Um gerade bei niedrigen CSB Werten eine ausreichende Messgenauigkeit zu erreichen, wird die Probe sowenig wie möglich verdünnt.
Zum Austreiben des Chlorids wird die Probe typischerweise in einem Volumenverhältnis Probe : Schwefelsäure zwischen 1:2 und 2:1 mit der Schwefelsäure gemischt. Arbeitet man entsprechend der DIN-Norm, werden 20 ml der Probe mit 25 ml konzentrierter Schwefelsäure gemischt. Typischerweise wird dann über 1 bis 3 Stunden, bevorzugt ca. 2 Stunden, gerührt. Bevorzugt wird auf das Reaktionsgefäß eine Absorberkerze mit Absorbermaterial wie z.B. Natronkalk aufgesetzt. Einzelheiten finden sich in der DIN 38409-41, Methode 41-2.

Typischerweise wird nach dem Austreiben nicht die gesamte Probenmenge zur weiteren Bestimmung des CSB verwendet sondern nur ein Teil. Dieser wird mit der Reagenzlösung vermischt. Bevorzugt erfolgt die Mischung in einem Volumenverhältnis Probe : Reagenzlösung zwischen 8:1 und 1:5. Besonders bevorzugt erfolgt die Mischung in einem Volumenverhältnis zwischen 5:1 und 2:1.

Die Mischung aus Reagenzlösung und Probe sollte einen Schwefelsäuregehalt zwischen 48 uns 65% (w/w) aufweisen.

Anschließend wird die Mischung inkubiert. Bevorzugt erfolgt die Inkubation für 20 bis 150 Minuten bei einer Temperatur zwischen 120 und 180°C.

Um Norm-konform zu arbeiten, sollte die chemische Oxidation der Bestimmung, d.h. die Inkubation, zwischen 145°C und 155°C, idealerweise bei ca. 150°C über ungefähr 2h erfolgen.

Um einen Verlust von in der Probe enthaltenen flüchtigen org. Verbindungen zu vermeiden, ist die chemische Oxidation in einem gasdicht verschlossenen Gefäß von großem Vorteil. Daher wird erfindungsgemäß bevorzugt eine reversibel verschließbare Küvette als Reaktionsgefäß eingesetzt.

Für die Photometrische Messung ist es erforderlich, eine klare ungetrübte Messlösung vorliegen zu haben. Hierzu sollten sich alle für die Reaktion benötigten Komponenten unter den Reaktionsbedingungen (Temperatur und Druck) im Reaktionsgemisch lösen. Wird eine Maskierung von möglicherweise im Gemisch verbliebenen Chlorids mit Quecksilber(II)sulfat durchgeführt, so bildet sich ein Niederschlag aus Quecksilber(II)chlorid.

Dieser setzt sich in der typischerweise vor der photometrischen Messung durchgeführten Abkühlphase ab, sodass er die photometrische Messung nicht stört.

Die photometrische Messung erfolgt typischerweise durch Messung der Extinktion bei einer Wellenlänge im Bereich von 300 - 700 nm. Geeignet hierfür sind alle Photometer, die für Messungen zwischen 300 nm und 700 nm geeignet sind. Die Formel zur Berechnung des CSB ist dem Fachmann bekannt und findet sich z.B. in der DIN ISO 15705.

Es wurde gefunden, dass das erfindungsgemäße Verfahren zusätzlich verbessert und besonders empfindlich gestaltet werden kann, wenn zur Austreibung des Chlorids und auch zur Herstellung der Reagenzlösung eine Schwefelsäure verwendet wird, deren CSB maximal 5 mg/l beträgt.

Durch Variation des Mischungsverhältnisses zwischen Probe und Reagenzlösung und/oder Messung bei unterschiedlichen Wellenlängen in dem oben angegebenen Bereich, kann der Fachmann das Messverfahren auf Proben mit sehr hohem oder sehr niedrigem CSB anpassen.

Somit stellt die vorliegende Erfindung erstmals ein photometrisches Verfahren zur Bestimmung des CSB zur Verfügung, mit dem auch Proben mit einem hoher Chlorid-Gehalt vermessen werden können. Weiterhin ermöglicht das Verfahren, die Bestimmung ohne das toxische und umweltschädliche Quecksilber(II)-sulfat durchzuführen.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiele

### Herstellung der Reagenzlösungen

Die Herstellung der Reagenzlösung erfolgt durch mischen von 0,3 ml Lösung A,
1,0 ml Lösung B und 0,3 ml Lösung C.
Lösung A durch Lösen von Quecksilber(II)sulfat 15,5% (w/w) in Schwefelsäure 14,5% (w/w) herstellen.

Lösung B durch Lösen von Kaliumdichromat 0,1% (w/w) in Wasser herstellen. Lösung C durch Lösen von Silbersulfat 4,3% (w/w) in Wasser herstellen.

### Geräte

- Reaktionsküvette aus Röhrenglas Schott Fiolax® klar, mit einem Aussendurchmesser von 16 mm und einem Innendurchmesser von 13,4 mm
- Filterphotometer oder Spektralphotometer mit Vorrichtung zur Messung der beschriebenen Reaktionsküvette bei Wellenlängen im Bereich von 300 - 700 nm.
- Thermoreaktoren zur Inkubation der beschriebenen Reaktionsküvette im Temperaturbereich von 100 - 180°C
- Magnetrührer mit einstellbarer Drehzahl von 100 - 400 U/min
- Absorberkerze für Chlorid-Abreicherung nach DIN 38409-41-2
- Schliff-Erlenmeyerkolben 300 ml für Chlorid-Abreicherung nach DIN 38409-41-2
- Absorbermaterial für Chlorid-Abreicherung nach DIN 38409-41-2 (z.B. Natronkalk)
- Glas- oder Kolbenhubpipetten 0,3 - 25,0 ml

### Durchführung des Verfahrens

- 20 ml Probe mit einer Glaspipette in einen Schliff-Erlenmeyerkolben 300 ml geben. Ein Magnetrührstab von 30-50 mm Länge hinzufügen. Anschließend mit einer Glaspipette langsam und unter Rühren 25 ml Schwefelsäure 96% (w/w) zugeben. Die Temperatur der Mischung soll unter 40°C liegen. Ggf. ist die Mischung in einem Eisbad zu kühlen.
- Eine Absorberkerze nach DIN 38409-42-2 mit einem geeigneten Absorbermaterial (z.B. 5 g Natronkalk) befüllen und auf den Schliff-Erlenmeyerkolben aufsetzen und mit einem Glasstopfen verschließen (Skizze siehe DIN 38409-42-2).
- Die beschriebene Apparatur auf einen Magnetrührer stellen und bei einer Drehzahl von 250 U/min für 2 Stunden rühren. Bei Proben mit einem Chloridgehalt von über 10 g/l wird empfohlen das Absorbermaterial in der Absorberkerze nach 1 Stunde auszutauschen.
- Während der Chlorid-Abreicherung die Reaktionsküvette mit der Reaktionslösung befüllen. Wird ein gebrauchsfertiger Testsatz benutzt, entfällt dieser Schritt.
- Nach der Chlorid-Abreicherung mit einer Glas- oder Kolbenhubpipette 5 ml der schwefelsauren Chlorid-abgereicherten Probe entnehmen und in eine vorbereitete Reaktionsküvette geben.
- Die Reaktionsküvette verschließen, mischen und 2 Stunden in einem vortemperierten Thermoreaktor bei 148°C inkubieren. Heiße Küvette aus dem Thermoreaktor nehmen und zum Abkühlen in ein Reagenzglasgestell stellen. Nach 10 Minuten Küvette umschwenken und zum Abkühlen auf Raumtemperatur in das Reagenzglasgestell zurückstellen.
- Die Messprobe in einem Photometer bei 340 nm messen.

### Auswertung

Die Auswertung erfolgt über eine im Photometer hinterlegte Kalibrierfunktion. Die Ermittlung der Kalibrierfunktion erfolgt nach ISO 8466-1 und DIN 38402 A51.

## Patentansprüche

1. Küvette enthaltend eine Reagenzlösung zumindest bestehend aus
- Kaliumdichromat (K₂Cr₂O₇)
- Silbersulfat (Ag₂SO₄)
- Wasser
- Schwefelsäure
**dadurch gekennzeichnet, dass** der Gehalt an Schwefelsäure zwischen 20 und 50 (w/w) beträgt, der Gehalt an Kaliumdichromat zwischen 0,1 und 2,5 % (w/w) liegt und der Gehalt an Silbersulfat zwischen 0,1 und 2 % (w/w) liegt.

2. Küvette nach Anspruch 1, **dadurch gekennzeichnet, dass** die Küvette reversibel verschließbar ist.

3. Küvette nach einem der Ansprüche 1 oder 2, **dadurch gekennzeichnet, dass** die Reagenzlösung zusätzlich Quecksilber(II)sulfat (HgSO₄) enthält.

4. Küvette nach einem oder mehreren der Ansprüche 1 bis 3, **dadurch gekennzeichnet, dass** die Reagenzlösung nur aus 0,1 bis 2,5 Gewichts% Kaliumdichromat (K₂Cr₂O₇), 0,1 bis 2 Gewichts% Silbersulfat (Ag₂SO₄), 20 bis 50 Gewichts% Schwefelsäure und Wasser besteht.

5. Küvette nach einem oder mehreren der Ansprüche 1 bis 4, **dadurch gekennzeichnet, dass** die Reagenzlösung hergestellt ist durch Mischen von mindestens einer Lösung, die zumindest Kaliumdichromat und Wasser, aber kein Silbersulfat und kein Quecksilber(II)sulfat enthält und einer Lösung, die zumindest Silbersulfat und Schwefelsäure, aber kein Quecksilbersulfat und kein Kaliumdichromat enthält.

6. Verfahren zur Bestimmung des chemischen Sauerstoffbedarfs in Chlorid-haltigen Proben durch
a) Austreiben der Chlorid-Ionen aus der Probe durch Behandlung mit Schwefelsäure
b) Mischen zumindest eines Teils der aus Schritt a) erhaltenen Probe mit einer Reagenzlösung zumindest bestehend aus
- Kaliumdichromat (K₂Cr₂O₇)
- Silbersulfat (Ag₂SO₄)
- optional Quecksilber(II)sulfat (HgSO₄)
- Wasser
- Schwefelsäure
**dadurch gekennzeichnet, dass** der Gehalt an Schwefelsäure zwischen 20 und 50 %(w/w) beträgt, der Gehalt an Kaliumdichromat zwischen 0,1 und 2,5 % (w/w) liegt und der Gehalt an Silbersulfat zwischen 0,1 und 2 % (w/w) liegt
c) Inkubation der Mischung aus Schritt b) für mindestens 20 Minuten bei einer Temperatur über 100°C.
d) photometrische Bestimmung des CSB Gehalts der Mischung aus c).

7. Verfahren nach Anspruch 6, **dadurch gekennzeichnet, dass** die Verfahrensschritte b) bis d) in einer reversibel verschließbaren Küvette durchgeführt werden.

8. Verfahren nach einem der Ansprüche 6 oder 7, **dadurch gekennzeichnet, dass** das Austreiben in Schritt a) mit konzentrierter Schwefelsäure erfolgt.

9. Verfahren nach einem oder mehreren der Ansprüche 6 bis 8, **dadurch gekennzeichnet, dass** die Inkubation in Schritt c) für 20 bis 150 Minuten bei einer Temperatur zwischen 120 und 180°C erfolgt.

10. Verfahren nach einem oder mehreren der Ansprüche 6 bis 9, **dadurch gekennzeichnet, dass** in Schritt b) die Probe und die Reagenzlösung in einem Volumenverhältnis Probe : Reagenzlösung zwischen 5:1 und 1:5 gemischt werden.

11. Verfahren nach einem oder mehreren der Ansprüche 6 bis 10, **dadurch gekennzeichnet, dass** die Reagenzlösung nur aus 0,1 bis 2,5 Gewichts% Kaliumdichromat (K₂Cr₂O₇), 0,1 bis 2 Gewichts% Silbersulfat (Ag₂SO₄), 20 bis 50 Gewichts% Schwefelsäure und Wasser besteht.

12. Verfahren nach einem oder mehreren der Ansprüche 6 bis 11, **dadurch gekennzeichnet, dass** die Reagenzlösung hergestellt ist durch Mischen von mindestens einer Lösung, die zumindest Kaliumdichromat und Wasser, aber kein Silbersulfat und kein Quecksilber(II)sulfat enthält und einer Lösung, die zumindest Silbersulfat und Schwefelsäure, aber kein Quecksilbersulfat und kein Kaliumdichromat enthält.

13. Verfahren nach einem oder mehreren der Ansprüche 6 bis 12, **dadurch gekennzeichnet, dass** die photometrische Bestimmung in Schritt c) durch Messung der Extinktion bei einer Wellenlänge im Bereich von 300 - 700 nm erfolgt.

14. Verfahren nach einem oder mehreren der Ansprüche 6 bis 13, **dadurch gekennzeichnet, dass** Schwefelsäure mit einem CSB von weniger als 5 mg/l eingesetzt wird.

15. Verfahren nach einem oder mehreren der Ansprüche 6 bis 13 , **dadurch gekennzeichnet, dass** für Schritt a) Schwefelsäure mit einem CSB von weniger als 5 mg/l verwendet wird.

## Claims

1. Cell containing a reagent solution at least consisting of
- potassium dichromate (K₂Cr₂O₇)
- silver sulfate (Ag₂SO₄)
- water
- sulfuric acid
**characterised in that** the sulfuric acid content is between 20 and 50% (w/w), the potassium dichromate content is between 0.1 and 2.5% (w/w) and the silver sulfate content is between 0.1 and 2% (w/w).

2. Cell according to Claim 1, **characterised in that** the cell can be sealed reversibly.

3. Cell according to one of Claims 1 or 2, **characterised in that** the reagent solution additionally contains mercury(II) sulfate (HgSO₄).

4. Cell according to one or more of Claims 1 to 3, **characterised in that** the reagent solution consists only of 0.1 to 2.5% by weight of potassium dichromate (K₂Cr₂O₇), 0.1 to 2% by weight of silver sulfate (Ag₂SO₄), 20 to 50% by weight of sulfuric acid and water.

5. Cell according to one or more of Claims 1 to 4, **characterised in that** the reagent solution is prepared by mixing at least one solution which contains at least potassium dichromate and water, but no silver sulfate and no mercury(II) sulfate, and a solution which contains at least silver sulfate and sulfuric acid, but no mercury sulfate and no potassium dichromate.

6. Method for the determination of the chemical oxygen demand in chloride-containing samples by
a) expulsion of the chloride ions from the sample by treatment with sulfuric acid
b) mixing of at least part of the sample obtained from step a) with a reagent solution at least consisting of
- potassium dichromate (K₂Cr₂O₇)
- silver sulfate (Ag₂SO₄)
- optionally mercury(II) sulfate (HgSO₄)
- water
- sulfuric acid
**characterised in that** the sulfuric acid content is between 20 and 50% (w/w), the potassium dichromate content is between 0.1 and 2.5% (w/w) and the silver sulfate content is between 0.1 and 2% (w/w)
c) incubation of the mixture from step b) for at least 20 minutes at a temperature above 100°C
d) photometric determination of the COD content of the mixture from c).

7. Method according to Claim 6, **characterised in that** method steps b) to d) are carried out in a cell which can be sealed reversibly.

8. Method according to one of Claims 6 or 7, **characterised in that** the expulsion in step a) is carried out using concentrated sulfuric acid.

9. Method according to one or more of Claims 6 to 8, **characterised in that** the incubation in step c) is carried out for 20 to 150 minutes at a temperature between 120 and 180°C.

10. Method according to one or more of Claims 6 to 9, **characterised in that** the sample and the reagent solution are mixed in step b) in a sample : reagent solution volume ratio between 5:1 and 1:5.

11. Method according to one or more of Claims 6 to 10, **characterised in that** the reagent solution consists only of 0.1 to 2.5% by weight of potassium dichromate (K₂Cr₂O₇), 0.1 to 2% by weight of silver sulfate (Ag₂SO₄), 20 to 50% by weight of sulfuric acid and water.

12. Method according to one or more of Claims 6 to 11, **characterised in that** the reagent solution is prepared by mixing at least one solution which contains at least potassium dichromate and water, but no silver sulfate and no mercury(II) sulfate, and a solution which contains at least silver sulfate and sulfuric acid, but no mercury sulfate and no potassium dichromate.

13. Method according to one or more of Claims 6 to 12, **characterised in that** the photometric determination in step c) is carried out by measurement of the absorbance at a wavelength in the range 300 - 700 nm.

14. Method according to one or more of Claims 6 to 13, **characterised in that** sulfuric acid having a COD of less than 5 mg/l is employed.

15. Method according to one or more of Claims 6 to 13 , **characterised in that** sulfuric acid having a COD of less than 5 mg/l is used for step a).

## Revendications

1. Cellule contenant une solution de réactifs au moins constituée par
- du dichromate de potassium (K₂Cr₂O₇)
- du sulfate d'argent (Ag₂SO₄)
- de l'eau
- de l'acide sulfurique
**caractérisée en ce que** la teneur en acide sulfurique est comprise entre 20 et 50% (p/p), la teneur en dichromate de potassium est comprise entre 0,1 et 2,5% (p/p) et la teneur en sulfate d'argent est comprise entre 0,1 et 2% (p/p).

2. Cellule selon la revendication 1, **caractérisée en ce que** la cellule peut être scellée de manière réversible.

3. Cellule selon l'une parmi les revendications 1 ou 2, **caractérisée en ce que** la solution de réactifs contient en outre du sulfate de mercure(II) (HgSO₄).

4. Cellule selon l'une ou plusieurs parmi les revendications 1 à 3, **caractérisée en ce que** la solution de réactifs n'est constituée que de 0,1 à 2,5% en poids de dichromate de potassium (K₂Cr₂O₇), de 0,1 à 2% en poids de sulfate d'argent (Ag₂SO₄), de 20 à 50% en poids d'acide sulfurique et d'eau.

5. Cellule selon l'une ou plusieurs parmi les revendications 1 à 4, **caractérisée en ce que** la solution de réactifs est préparée par le mélange d'au moins une solution qui contient au moins du dichromate de potassium et de l'eau, mais aucun sulfate d'argent et aucun sulfate de mercure(II), et une solution qui contient au moins du sulfate d'argent et de l'acide sulfurique, mais aucun sulfate de mercure et aucun dichromate de potassium.

6. Méthode de détermination de la demande chimique en oxygène dans des échantillons contenant des chlorures, par
a) l'expulsion des ions chlorure à partir de l'échantillon par un traitement par de l'acide sulfurique
b) le mélange d'au moins une partie de l'échantillon obtenu dans l'étape a) avec une solution de réactifs au moins constituée par
- du dichromate de potassium (K₂Cr₂O₇)
- du sulfate d'argent (Ag₂SO₄)
- éventuellement du sulfate de mercure(II) (HgSO₄)
- de l'eau
- de l'acide sulfurique
**caractérisée en ce que** la teneur en acide sulfurique est comprise entre 20 et 50% (p/p), la teneur en dichromate de potassium est comprise entre 0,1 et 2,5% (p/p) et la teneur en sulfate d'argent est comprise entre 0,1 et 2% (p/p)
c) l'incubation du mélange de l'étape b) pendant au moins 20 minutes à une température supérieure à 100°C
d) la détermination photométrique de la teneur en DCO du mélange issu de c).

7. Méthode selon la revendication 6, **caractérisée en ce que** les étapes de méthode b) à d) sont mises en œuvre dans une cellule qui peut être scellée de manière réversible.

8. Méthode selon l'une parmi les revendications 6 ou 7, **caractérisée en ce que** l'expulsion dans l'étape a) est mise en œuvre à l'aide d'acide sulfurique concentré.

9. Méthode selon l'une ou plusieurs parmi les revendications 6 à 8, **caractérisée en ce que** l'incubation dans l'étape c) est mise en œuvre pendant de 20 à 150 minutes à une température comprise entre 120 et 180°C.

10. Méthode selon l'une ou plusieurs parmi les revendications 6 à 9, **caractérisée en ce que** l'échantillon et la solution de réactifs sont mélangés dans l'étape b) selon un rapport de volume échantillon : solution de réactifs compris entre 5:1 et 1:5.

11. Méthode selon l'une ou plusieurs parmi les revendications 6 à 10, **caractérisée en ce que** la solution de réactifs n'est constituée que de 0,1 à 2,5% en poids de dichromate de potassium (K₂Cr₂O₇), de 0,1 à 2% en poids de sulfate d'argent (Ag₂SO₄), de 20 à 50% en poids d'acide sulfurique et d'eau.

12. Méthode selon l'une ou plusieurs parmi les revendications 6 à 11, **caractérisée en ce que** la solution de réactifs est préparée par le mélange d'au moins une solution qui contient au moins du dichromate de potassium et de l'eau, mais aucun sulfate d'argent et aucun sulfate de mercure(II), et une solution qui contient au moins du sulfate d'argent et de l'acide sulfurique, mais aucun sulfate de mercure et aucun dichromate de potassium.

13. Méthode selon l'une ou plusieurs parmi les revendications 6 à 12, **caractérisée en ce que** la détermination photométrique dans l'étape c) est mise en œuvre par la mesure de l'absorbance à une longueur d'onde dans la plage de 300 - 700 nm.

14. Méthode selon l'une ou plusieurs parmi les revendications 6 à 13, **caractérisée en ce qu'**on emploie de l'acide sulfurique ayant une DCO inférieure à 5 mg/l.

15. Méthode selon l'une ou plusieurs parmi les revendications 6 à 13 , **caractérisée en ce qu'**on utilise de l'acide sulfurique ayant une DCO inférieure à 5 mg/l pour l'étape a).
